# FASCICULE DE BREVET EUROPEEN

(11) **EP 1 458 298 B1**
(45) Date de publication et mention de la délivrance du brevet: **08.09.2010**
(21) Numéro de dépôt: 02791886.1
(22) Date de dépôt: 04.10.2002
(51) Int. Cl.: A61B 17/70, A61B 17/88

(54) **ENSEMBLE POUR L'OSTEOSYNTHESE DU RACHIS COMPRENANT UNE TETE D'ORGANE D'ANCRAGE ET UN OUTIL POUR LA FIXATION DE LA TETE**
SPINALE OSTEOSYNTHESE-ANORDNUNG MIT DEM KOPF EINES VERANKERUNGSELEMENTS UND EINEM WERKZEUG ZUR FIXIERUNG DIESES KOPFES
SPINAL OSTEOSYNTHESIS ASSEMBLY COMPRISING THE HEAD OF AN ANCHORING MEMBER AND A TOOL FOR FIXING SAID HEAD

(30) Priorité: 04.10.2001 FR 0112754
(43) Date de publication de la demande: 22.09.2004
(73) Titulaire: Stryker Spine, 33610 Cestas (FR)
(72) Inventeur: SAINT MARTIN, Pierre Henri, F-33700 Merignac (FR); VIENNEY, Cécile, F-33000 Bordeaux (FR); DE CONINCK, Cédric, F-33610 Cestas Gazinet (FR)
(74) Mandataire: Le Forestier, Eric
(86) Numéro de dépôt international: PCT/FR2002/003391
(87) Numéro de publication internationale: WO 2003/028566

(56) Documents cités:
- US-A- 4 411 259
- US-A- 5 720 751
- US-A- 6 036 692
- US-B1- 6 183 472

## Description

L'invention concerne les systèmes d'ostéosynthèse du rachis et en particulier les outils utilisés durant la fixation de ces systèmes.

On connaît de nombreux systèmes d'ostéosynthèse du rachis, par exemple le système divulgué dans le document EP-0 885 598. Ces systèmes comprennent généralement des organes d'ancrages sous la forme de vis ou de crochets reliés entre eux par un ou plusieurs éléments de liaison sous la forme de tiges ou de plaques. Ces systèmes ont pour fonction de rectifier la forme d'une colonne vertébrale déformée ou d'assurer l'ostéosynthèse d'une ou plusieurs vertèbres fracturées. Les organes d'ancrage sont à cette fin rigidement fixés aux vertèbres.

Ces organes doivent être placés chacun dans une position très précise. On connaît pour cela des outils permettant de tenir provisoirement l'organe en vue de l'assembler rigidement aux autres pièces dans la position souhaitée. Toutefois, la plupart de ces outils ne donnent pas satisfaction, soit qu'ils n'assurent pas une tenue satisfaisante de l'organe ou qu'ils nécessitent en raison de leur encombrement de relâcher l'organe en vue de le fixer aux autres éléments du système. En particulier, ces outils ne donnent pas satisfaction lorsqu'il s'agit de tenir un organe d'ancrage dont la tête comprend au moins une branche.

Un but de l'invention est de fournir un outil permettant de tenir convenablement la tête d'un organe d'ancrage comprenant au moins une branche, même durant la fixation de cet organe à d'autres parties du système.

A cet effet, on prévoit un ensemble pour l'ostéosynthèse du rachis comprenant :
- une tête d'organe d'ancrage comportant au moins une branche ; et
- un outil pour la fixation de la tête à un système d'ostéosynthèse du rachis,
   l'outil étant apte à tenir la tête en réalisant au moins un appui latéral sur au moins un bord latéral de la branche et un appui supplémentaire sur la tête en direction opposée ou à chaque appui latéral.

Ainsi l'appui sur au moins l'un des deux bords latéraux rend possible de réaliser l'appui supplémentaire ailleurs que sur la face de la branche destinée à venir en contact avec l'élément de liaison. L'appui supplémentaire peut par exemple être réalisé au sommet de la branche ou sur l'autre face de la branche. La tête peut donc être assemblée aux autres éléments du système tandis qu'elle est tenue par l'outil. De plus, l'outil présente deux voire trois points d'appui avec la tête, ce qui assure une tenue correcte de la tête par l'outil.

L'ensemble selon l'invention pourra en outre présenter au moins l'une quelconque des caractéristiques suivantes :
- l'outil est apte à réaliser deux appuis latéraux sur des bords latéraux respectifs de la branche,
- l'outil comprend un bras d'une seule pièce apte à réaliser simultanément les appuis latéraux et l'appui supplémentaire,
- l'outil et les bords latéraux sont conformés pour interdire par complémentarité de formes un glissement de l'outil vers le haut et/ou vers le bas de la tête,
- l'outil présente deux reliefs pour prendre appui sur les bords,
- les bords latéraux présentent deux cavités sur lesquelles l'outil est apte à prendre appui,
- les cavités sont débouchantes en direction d'un sommet de la tête,
- un appui supplémentaire est réalisé sur un bord supérieur de la branche,
- le contact de l'outil avec le bord supérieur est linéique ou surfacique,
- l'outil comprend un élément allongé s'étendant transversalement par référence à une direction générale de l'outil et par lequel il est apte à prendre appui sur le bord supérieur,
- l'outil comprend deux doigts supportant l'élément allongé, l'ensemble étant agencé de sorte que l'élément allongé peut être disposé dans un logement de la tête sans que les doigts s'étendent dans le logement,
- les doigts s'étendent à une extrémité d'un bras de l'outil,
- l'outil comprend un bras apte à réaliser l'appui supplémentaire,
- l'appui supplémentaires est réalisé sur une face de la branche,
- l'outil et la face sont conformés pour interdire par complémentarité de formes un glissement de l'outil vers le haut et/ou vers le bas de la tête,
- la branche étant une première branche, la tête comporte une deuxième branche s'étendant en regard de la première branche.
- le ou chaque appui latéral est réalisé en direction opposée à la deuxième branche,
- l'outil est apte à tenir la branche en laissant accessible un logement formé entre les deux branches,
- l'outil comprend deux bras articulés l'un à l'autre,
- l'ensemble comprend un organe d'ancrage apte à être ancré dans le rachis, la tête étant d'un seul tenant avec cet organe,
- l'ensemble comprend un organe d'ancrage apte à être ancré dans le rachis, la tête étant apte à être fixée à l'organe suivant différentes positions angulaires de la tête par rapport à l'organe, et
- système d'ostéosynthèse du rachis comprenant un organe d'ancrage comportant une tête, la tête comportant une branche présentant au moins un bord latéral, le ou chaque bord présentant une cavité borgne entament ce bord.

D'autres caractéristiques et avantages de l'invention apparaîtront encore dans la description qui va suivre d'un mode préféré de réalisation et de variantes donnés à titre d'exemples non limitatifs. Aux dessins annexées :
- la figure 1 est une vue en perspective d'une tête d'un organe d'ancrage d'un ensemble selon le mode préféré de réalisation de l'invention ;
- la figure 2 est une vue à plus grande échelle d'un organe d'ancrage avec sa tête, selon une variante de réalisation ;
- la figure 3 est une vue partielle en perspective d'un outil de l'ensemble selon le mode préféré de réalisation de l' invention ;
- les figures 4 à 7 sont quatre vues en perspective illustrant quatre étapes de la coopération de l'outil de la figure 3 avec la tête de la figure 1 au cours de l'assemblage de l'organe d'ancrage avec les autres éléments du système d'ostéosynthèse ; et
- les figures 8 et 9 sont deux vues analogues à la figure 1 illustrant des variantes de réalisation de la tête.

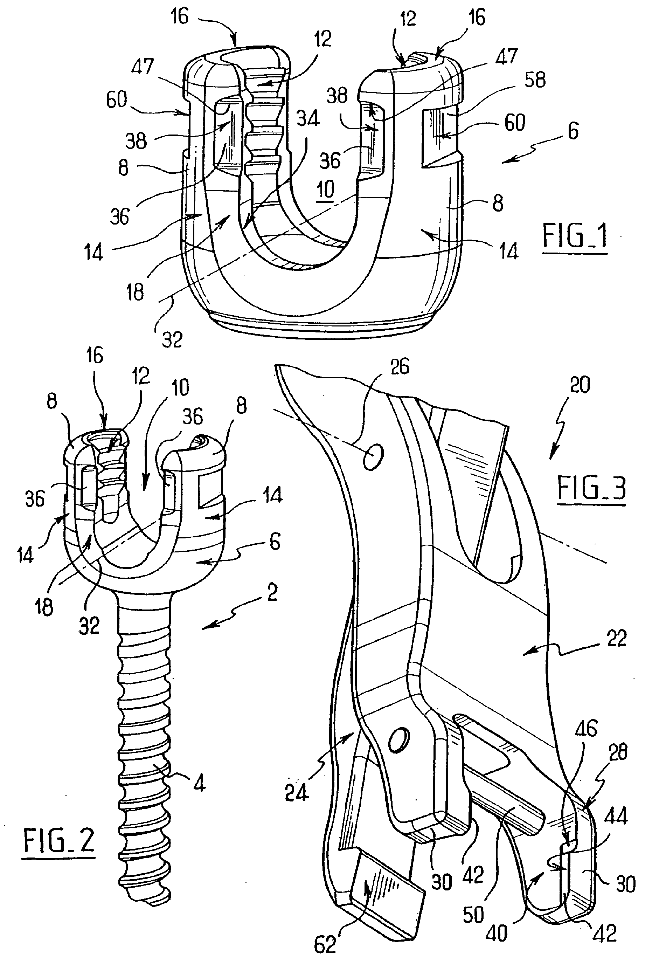

On va décrire un mode préféré de réalisation de l'ensemble pour l'ostéosynthèse du rachis. Cet ensemble comprend un outil illustré à la figure 3 et un système d'ostéosynthèse du rachis. Ce système comprend de nombreuses pièces parmi lesquelles des organes d'ancrage identiques entre eux, du type de ceux des figures 1 et 2.

Chaque organe comprend un corps fileté et une tête. Dans le mode de la figure 1, la tête fait partie d'un organe d'ancrage de type polyaxial connu en soi. Dans un tel organe, la tête 6 peut être montée sur le corps de l'organe en occupant différentes positions angulaires par rapport à celui-ci et être immobilisée dans la position angulaire choisie. La tête 6 comprend deux branches 8 s'étendant à distance et en regard l'une de l'autre. Les deux branches donnent à la tête une forme en U en définissant entre elles un logement 10. L'organe a dans son ensemble une forme de diapason identique à celle de la variante de la figure 2. Les deux branches 8 ont une forme en secteur de cylindre défini géométriquement par un même cylindre coaxial au corps 4. Les deux branches sont symétriques l'une de l'autre par rapport à un plan longitudinal médian formant plan de symétrie de l'organe. L'organe présente par ailleurs un autre plan longitudinal médian de symétrie perpendiculaire au premier.

Chaque branche 8 présente une face interne 12 faisant face à l'autre branche et une face externe 14 opposée à l'autre branche. Chaque branche présente un sommet 16 essentiellement plan et en forme d'arc de cercle, et deux bords latéraux 18 formant chacun la jonction entre les faces interne et externe de la branche. Ces caractéristiques de l'organe d'ancrage 2 sont connues en elles-mêmes et ne seront pas décrites plus en détail ici. On pourra notamment se référer au document EP-0 885 598.

En référence notamment à la figure 3, l'outil 20 de l'ensemble selon l'invention comprend deux bras 22 et 24 ayant chacun une forme générale allongée sensiblement rectiligne. Les deux bras sont articulés l'un à l'autre suivant un axe 26 perpendiculaire à la direction longitudinale des bras. Le bras 22 a suivant cet axe 26 une largeur plus grande que la largeur de l'autre bras 24. Nous appellerons ici premier bras, le bras 22. Le premier bras 22 a une forme de fourche sur une grande partie de sa longueur s'étendant depuis une extrémité proximale non illustrée du bras jusqu'à une zone du bras se trouvant entre l'axe 26 et l'extrémité distale 28 du bras. Cette extrémité distale 28 présente elle aussi une forme de fourche quoique sur une longueur beaucoup plus courte que l'autre fourche. Cette fourche définit ainsi deux doigts 30 qui forment à eux deux l'extrémité distale 28. Le deuxième bras 24 traverse la grande fourche du premier bras au niveau de l'axe 26.

La tête 6 et l'outil 22 sont configurés pour que l'outil soit apte à tenir la tête, notamment de façon rigide. On va maintenant décrire les moyens permettant cette tenue.

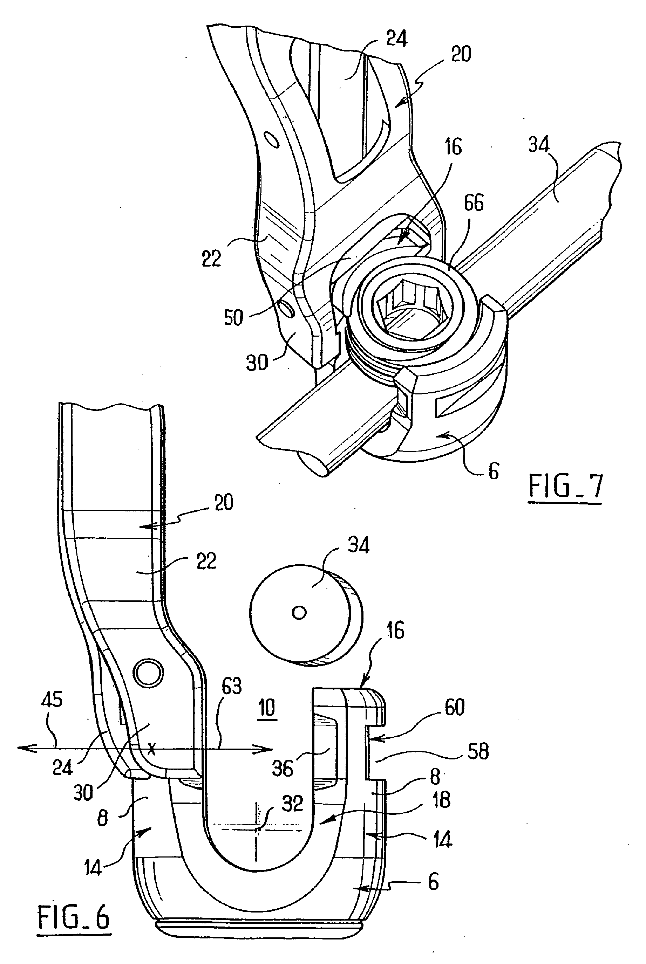

Sur la tête 6, chaque bord 18 présente un chanfrein s'étendant depuis le sommet du bord jusqu'en partie basse de la tête où il se prolonge en continuité avec le bord correspondant de l'autre branche. Chaque paire de bords forme donc elle aussi un U. Le chanfrein est orienté sensiblement vers l'extérieur de la tête, essentiellement suivant une direction parallèle à un axe 32 suivant lequel est destiné à s'étendre une tige de liaison 34 telle qu'illustrée à la figure 7, dans le logement défini entre les branches.

La tête 6 présente des deuxièmes chanfreins 36 s'étendant à la jonction de chaque bord 18 avec les faces internes 12 des branches. Ce deuxième chanfrein 34 a lui aussi une forme générale de U. Il est essentiellement dirigé vers l'intérieur de la tête. Il a en partie basse une forme essentiellement cylindrique d'axe 32.

Chaque bord latéral 18 de chaque branche présente une cavité borgne 36 formée à la fois dans le bord 18 et le deuxième chanfrein 36 en entamant l'arrête formée à la jonction de ceux-ci. Chaque cavité 36 présente une face de fond plane 38 qui constitue la plus grande face de la cavité. La cavité débouche latéralement respectivement sur le bord 18 et le deuxième chanfrein 36. En revanche, elle est obturée vers le haut et vers le bas par les parties résiduelles de l'arête. La face 38 est parallèle à l'axe du cylindre formant les branches. Elle est inclinée par rapport à l'axe 32 et orientée vers l'extérieur de la tête. Elle est orientée essentiellement en direction opposée à la partie de la branche qui la porte. La tête 6 présente ainsi quatre cavités 36, deux par branche 8.

Les doigts 30 présentent deux faces planes 40 s'étendant parallèlement l'une à l'autre, en regard l'une de l'autre et à distance l'une de l'autre. Chaque doigt comporte un relief 42 s'étendant en saillie de la face 40 en direction de l'autre doigt. Ce relief est contigu à un bord avant du doigt 30. Chaque relief 42 présente en particulier une face plane 44 parallèle à une direction longitudinale de l'outil et orientée vers l'autre doigt tout en étant inclinée vers l'arrière, c'est-à-dire en direction du deuxième bras 24 lorsque ceux-ci sont dans la position de la figure 3 où les extrémités distales des deux bras sont voisines l'une de l'autre.

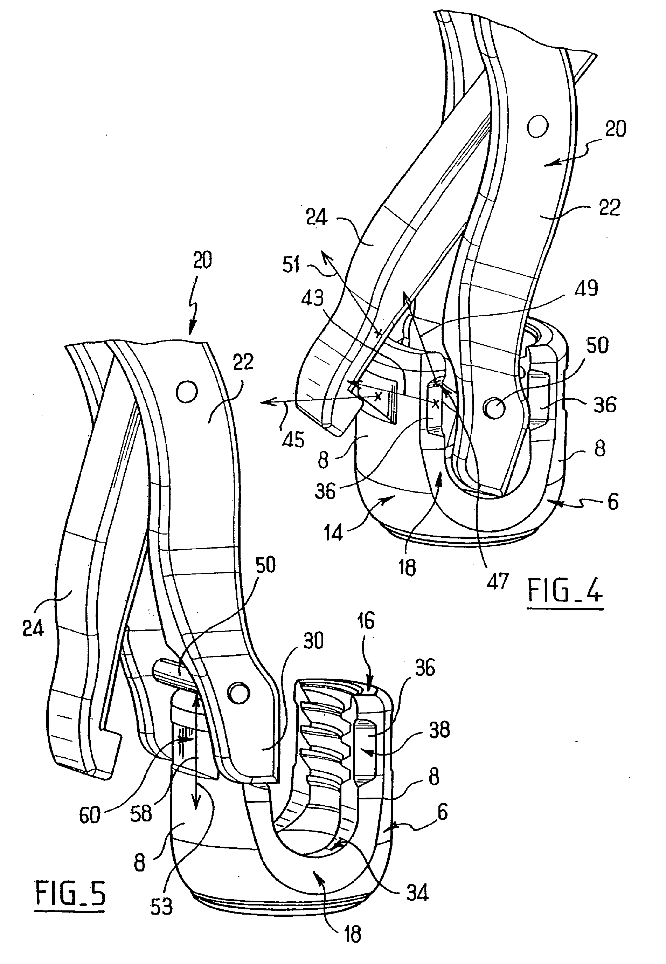

Les cavités 36 et les reliefs 42 sont conformés et positionnés de sorte que le premier bras 22 peut être disposé par rapport à la tête 6 afin que les faces 44 des reliefs 42 viennent en contact avec les faces 38 des deux cavités 36 respectives de l'une quelconque des deux branches. Dans cette position, illustrée notamment aux figures 5, 6 et 7, chacun des reliefs 42 réalise sur la cavité 36 associée un appui latéral 43 orienté en direction opposée à l'autre branche (un seul de ces appuis est illustré à la figure 4). La résultante 45 de ces deux appuis forme un appui orienté en direction opposée à l'autre branche.

Chaque relief 42 présente par ailleurs une face supérieure 46 de forme plane, inclinée par rapport à la direction verticale et formant un pan coupé de ce relief.

Lorsque le premier bras 22 est en appui sur les bords 18 comme on vient de l'expliquer, les faces 46 des reliefs sont en appui contre les faces supérieures 47 des cavités 36 respectives. L'appui latéral correspondant 49 est lui aussi orienté en direction opposée à l'autre branche mais est surtout orienté vers le haut en direction du sommet de la branche. Ici encore la résultante 51 de ces deux appuis forme un appui orienté en direction opposée à l'autre branche et vers le haut.

En référence à la figure 3, l'outil 20 comprend un élément allongé transversal 50 réalisé ici sous la forme d'une tige. La tige 50 s'étend parallèlement à l'axe de rotation 26, de l'un à l'autre des doigts 30 dans la fourche définie par ceux-ci.

La tige 50 est conformée et positionnée de sorte que, lorsque les reliefs 42 sont en appui dans les cavités 36 comme expliqué plus haut, la tige 50 vient en appui sur le sommet 16 de la même branche comme illustré notamment aux figures 5 et 7. L'appui 58 de la tige 50 sur ce sommet est orienté vers le bas de la tête parallèlement à la direction longitudinale de l'organe d'ancrage comme illustré à la figure 5. Compte tenu de la forme cylindrique de la face extérieure de la tige et de la forme essentiellement plane du sommet 16, le contact entre ces deux éléments est linéique. En présence d'un élément transversal 50 à section droite rectangulaire ou carrée, ce contact serait surfacique.

Si l'on considère l'appui 51 résultant des appuis 49 des faces 46 sur les faces 47 des cavités comme un premier appui et l'appui 58 de la tige 50 sur le sommet 16 comme un deuxième appui, on voit que ces deux appuis sont orientés dans des directions opposées même si ces directions ne sont pas rigoureusement colinéaires. Toutefois, lorsque l'outil tient la tête par ces seuls appuis, il faut tenir compte de l'action de la gravité sur la tête. On voit dans la position de la figure 5 que la ligne imaginaire formée par les deux faces 46 est très proche de la tige 50 tandis que le centre de gravité de la tête est éloigné de celle-ci. En conséquence, si l'on considère que la tête est mobile à rotation autour de la ligne formée par les deux faces 46, le poids de la tête tend à la faire tourner de façon à plaquer le sommet 16 contre la tige 50, aboutissant ainsi à une position stable de la tête tenue par l'outil. Bien que dans cette position la tête ne soit pas tenue rigidement par l'outil, elle peut être manipulée sans difficulté par l'intermédiaire de ce dernier notamment pour être déplacée.

La face externe 14 de chaque branche 8 présente une cavité 58 dont le fond est formé par un méplat 60 s'étendant parallèlement à l'axe 32 et à l'axe du cylindre. La face 60 a une forme généralement rectangulaire. Elle débouche latéralement sur la face externe 14 des deux cotés. Toutefois, elle est obturée vers le haut et vers le bas par les bords supérieur et inférieur de la cavité.

L'extrémité distale du deuxième bras 24 est orientée en direction de l'extrémité distale 28 du premier bras et se termine par une face plane 62 de forme rectangulaire sensiblement identique à celle des faces 60. La face 62 s'étend en direction des faces 44 des reliefs 42.

Les cavités 58 et la face 62 sont formées et positionnées de sorte que, lorsque le premier bras 22 est en appui sur l'une quelconque des branches au moyen des reliefs 42 et de la tige 50 comme on l'a expliqué plus haut, le deuxième bras 24 peut être manoeuvré pour faire pénétrer son extrémité proximale dans la cavité 58 de la même branche et ainsi mettre la face 62 en appui contre la face 60. L'appui 63 du deuxième bras sur la branche, illustré à la figure 6, est donc orienté en direction de l'autre branche et en direction opposée à l'appui 45 réalisé par le premier bras au moyen des reliefs 42. Si cette fois, on considère les deux appuis 43 formés par les reliefs 42 dans les cavités 36 comme un premier appui 45 et l'appui formé par la face 62 dans la cavité 60 comme un deuxième appui 63, on voit que ces deux appuis sont orientés en directions opposées et réalisent une sorte de pincement de la branche au moyen de l'outil. Ce pincement permet à l'opérateur de tenir rigidement la tête au moyen de l'outil. A cette fin, les cavités 58 sont de préférence à la hauteur des cavités 36. Cette tenue sera d'autant plus robuste que l'opérateur serrera en les rapprochant l'une de l'autre les extrémités proximales des bras. Cette tenue rigide de la tête au moyen de l'outil s'effectue par une seule des deux branches de la tête. Il peut s'agir de l'une quelconque des deux branches.

On remarquera par ailleurs que la tenue de la tête au moyen seulement des reliefs 42 et de la tige 50 par l'outil a lieu au moyen du seul premier bras 22.

La configuration de l'extrémité du premier bras 22 est telle que la tige 50 peut être disposée dans le logement 10 comme illustré à la figure 4 sans que les doigts 30 s'étendent dans le logement. Dans cette situation, les doigts s'étendent alors à la verticale des bords 18. Lorsque la tête est supportée de façon mobile sur le corps de l'organe, cette position de l'outil dans la tête permet de faire tourner la tête autour de son axe par appui de la tige contre les branches. De même, lorsque la tête est d'un seul tenant avec le corps, cette position permet de faire tourner l'organe autour de son axe longitudinal.

On a illustré aux figures 4 à 6 différentes positions relatives de la tête et de l'outil qui peuvent apparaître au cours d'une intervention chirurgicale pour l'installation du système d'ostéosynthèse.

La figure 4 montre comme on vient de l'expliquer la disposition de la tige dans le logement 10 de la tête qui permet, au moyen d'une rotation de l'outil autour de son axe longitudinal, de faire tourner la tête ou l'organe autour de son propre axe jusqu'à atteindre la position souhaitée.

La figure 5 montre une tenue de la tête au moyen du seul premier bras 22 grâce aux reliefs 42 et à la tige 50. Le deuxième bras 24 est alors éloigné de la tête. En pratique, on peut passer directement de la position de la figure 4 à celle de la figure 5.

On remarquera que, dans la position de la figure 4, l'extrémité distale 28 du premier bras 22 assure un auto-centrage de l'outil par rapport à la tête avant de passer à la position de la figure 5.

En ramenant ensuite l'extrémité distale du deuxième bras 24 contre la branche déjà tenue, on assure un maintien rigide de la tête par l'outil comme illustré à la figure 6. La tête peut alors être fermement maintenue.

On remarquera que l'outil laisse entièrement dégagé le logement 10 entre les branches ainsi que l'accès à celui-ci permettant de cette façon d'installer un organe de liaison tel qu'une tige 34 dans ce logement.

L'étape suivante peut ensuite être en référence à la figure 7 l'installation d'un élément de verrouillage 66. Cet élément a ici la forme d'une bague filetée extérieurement de façon à coopérer avec un filetage ménagé dans la face interne 12 des branches pour que cette bague puisse venir serrer la tige 34 contre le fond du logement 10. L'agencement de l'organe d'ancrage, de la tige, et de la bague est connu en soi. La bague présente un orifice à six pans en son centre pour son serrage. L'outil permet le maintien de la tête en position éventuellement jusqu'à l'issue du serrage de la bague et l'immobilisation complète de l'organe d'ancrage par rapport à la tige. L'opérateur peut alors mettre fin à la tenue de la tête par l'outil. On comprend naturellement que l'outil n'est pas destiné à rester sur le système après l'intervention, l'outil ne faisant pas partie du système d'ostéosynthèse proprement dit.

L'outil selon ce mode de réalisation de l'invention permet donc de tenir la tête de deux façons d'une part au moyen du seul bras 22 et d'autre part rigidement au moyen des deux bras. Comme on peut le voir, dans chaque position de tenue de la tête par l'outil, le contact s'effectue en trois points au moins.

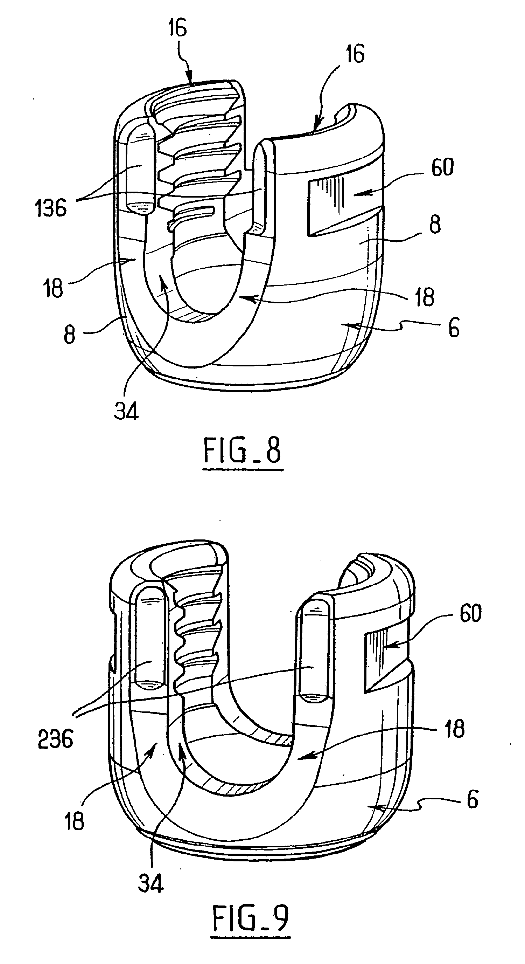

On a illustré à la figure 8 une variante de réalisation de l'invention. Dans cette variante, l'ensemble est identique à celui qui vient d'être décrit sauf que les cavités 136 ménagées dans les bords 18 de la tête débouchent vers le haut en direction du sommet des branches. Il s'ensuit que l'extrémité distale du premier bras 22 peut se séparer de la branche en coulissant vers le haut. En revanche, le maintien de la tête sur le premier bras 22 au moyen de la tige 50 sans intervention du deuxième bras 24, n'est plus possible.

Une autre variante de réalisation est illustrée à la figure 9. Dans ce mode de réalisation, la face de fond des cavités 236 a une forme cylindrique, l'axe du cylindre étant parallèle à la direction longitudinale de l'organe d'ancrage. A nouveau les cavités sont débouchantes vers le haut.

Lorsque la tête est d'une seule pièce avec le corps comme dans la variante de l'organe d'ancrage illustrée sur la figure 2, le chanfrein 34 peut être omis.

Bien entendu, on pourra apporter à l'invention de nombreuses modifications sans sortir du cadre de celle-ci.

On pourra prévoir que, durant la tenue rigide de la tête par pincement de la branche par les deux bras, le premier bras 22 réalise seulement un appui latéral 43 ou 49 dans une seule cavité 36, sous réserve de configurer de façon adaptée cette cavité et la face 58 pour que les deux appuis soient sensiblement en directions opposées. On pourra par exemple donner une forme non plane aux faces 60, 62 (par exemple sphérique) pour qu'elles s'assemblent suivant un montage mâle-femelle.

On pourra prévoir un outil muni d'un élément transversal 50 indépendamment des autres caractéristiques de l'invention.

## Revendications

1. Ensemble pour l'ostéosynthèse du rachis comprenant :
- une tête (6) d'organe d'ancrage (4) comportant au moins une branche (8) présentant une face interne (12), une face externe (14), un sommet (16) et deux bords latéraux (18); et
- un outil (20) pour la fixation de la tête (6) à un système d'ostéosynthèse du rachis,
**caractérisé en ce que** l'outil (20) étant agencé de sorte ä pouvoir tenir la tête (6)
en réalisant un appui latéral (43, 49) sur chacun des bords latéraux (18) de ladite branche et un appui supplémentaire (58) sur ladite branche en direction opposée à l'appui latéral, et **caractérisé**
**en ce que** l'outil (20) comprend un bras (22) d'une seule pièce apte à réaliser simultanément les appuis latéraux (43, 49) et l'appui supplémentaire (58).

2. Ensemble selon la revendication 1, **caractérisé en ce que** l'outil (20) et les bords latéraux (18) sont conformés pour interdire par complémentarité de formes un glissement de l'outil vers le haut et/ou vers le bas de la tête (6).

3. Ensemble selon la revendication 1 ou 2 **caractérisé en ce que** l'outil (20) présente deux reliefs (42) pour prendre appui sur les bords (18).

4. Ensemble selon l'une quelconque des revendications 1 à 3, **caractérisé en ce que** les bords latéraux (18) présentent deux cavités (36) sur lesquelles l'outil (20) est apte à prendre appui.

5. Ensemble selon la revendication 3, **caractérisé en ce que** les cavités (136; 236) sont débouchantes en direction d'un sommet (16) de la tête.

6. Ensemble selon l'une quelconque des revendications 1 à 5, **caractérisé en ce que** l'appui supplémentaire (58) est réalisé sur un bord supérieur (16) de la branche (8).

7. Ensemble selon la revendication 6, **caractérisé en ce que** le contact de l'outil (20) avec le bord supérieur (16) est linéique ou surfacique.

8. Ensemble selon l'une quelconque des revendications 6 ou 7, **caractérisé en ce que** l'outil (20) comprend un élément allongé (50) s'étendant transversalement par référence à une direction générale de l'outil et par lequel il est apte à prendre appui sur le bord supérieur (16).

9. Ensemble selon la revendication 8, **caractérisé en ce que** l'outil (20) comprend deux doigts (30) supportant l'élément allongé (50), l'ensemble étant agencé de sorte que l'élément allongé peut être disposé dans un logement (10) de la tête sans que les doigts (30) s'étendent dans le logement (10).

10. Ensemble selon la revendication 9, **caractérisé en ce que** les doigts (30) s'étendent à une extrémité d'un bras (22) de l'outil (20).

11. Ensemble selon l'une quelconque des revendications 1 à 10, **caractérisé en ce que** l'outil (20) comprend un bras (24) apte à réaliser l'appui supplémentaire (63).

12. Ensemble selon l'une quelconque des revendications 1 à 11, **caractérisé en ce que** l'appui supplémentaire (63) est réalisé sur une face (14) de la branche (8).

13. Ensemble selon la revendication 12, **caractérisé en ce que** l'outil (20) et la face (14) sont conformés pour interdire par complémentarité de formes un glissement de l'outil (20) vers le haut et/ou vers le bas de la tête (6).

14. Ensemble selon l'une quelconques des revendications 1 à 13, **caractérisé en ce que**, la branche (8) étant une première branche, la tête comporte une deuxième branche (8) s'étendant en regard de la première branche.

15. Ensemble selon la revendication 14, **caractérisé en ce que** le ou chaque appui latéral (43, 59) est réalisé en direction opposée à la deuxième branche (8).

16. Ensemble selon l'une quelconque des revendications 14 ou 15, **caractérisé en ce que** l'outil (20) est apte à tenir la branche (8) en laissant accessible un logement (10) formé entre les deux branches.

17. Ensemble selon l'une quelconque des revendications 1 à 16, **caractérisé en ce que** l'outil (20) comprend deux bras (22, 24) articulés l'un à l'autre.

18. Ensemble selon l'une quelconque des revendications 1 à 17, **caractérisé en ce qu'**il comprend un organe d'ancrage apte à être ancré dans le rachis, la tête (6) étant d'un seul tenant avec cet organe.

19. Ensemble selon l'une quelconque des revendications 1 à 17, **caractérisé en ce qu'**il comprend un organe d'ancrage apte à être ancré dans le rachis, la tête (6) étant apte à être fixée à l'organe suivant différentes positions angulaires de la tête par rapport à l'organe.

## Claims

1. A system for osteosynthesis of the spine, the system comprising:
- a head (6) of anchor member (4), the head including at least one branch (8) presenting an inside face (12), an outside face (14), a tip (16), and two lateral edges (18); and
- a tool (20) for fixing the head (6) to an assembly for osteosynthesis of the spine,
the tool (20) being arranged so as to hold the head (6) by applying lateral thrust (43, 49) on each lateral edge (18) of said branch and by applying additional thrust (58) on said branch in the opposite direction to the lateral thrust, and the system being **characterized in that** the tool (20) comprises a one-piece arm (22) suitable for applying the lateral thrust (43, 49) and the additional thrust (58) simultaneously.

2. A system according to claim 1, **characterized in that** the tool (20) and the lateral edges (18) are shaped with complementary shapes to prevent the tool sliding upwards and/or downwards relative to the head (6).

3. A system according to claim 1 or claim 2, **characterized in that** the tool (20) presents two portions in relief (42) for applying thrust against the edges (18).

4. A system according to any one of claims 1 to 3, **characterized in that** the lateral edges (18) present two cavities (36) against which the tool (20) is suitable for applying thrust.

5. A system according to claim 3, **characterized in that** the cavities (136; 236) are open towards the tip (16) of the head.

6. A system according to any one of claims 1 to 5, **characterized in that** the additional thrust (58) is applied to a top edge (16) of the branch (8).

7. A system according to claim 6, **characterized in that** the contact between the tool (20) and the top edge (16) takes place along a line or over an area.

8. A system according to claim 6 or claim 7, **characterized in that** the tool (20) includes an elongate element (50) extending transversely relative to a general direction of the tool, whereby the tool is capable of applying thrust to the top edge (16).

9. A system according to claim 8, **characterized in that** the tool (20) includes two fingers (30) supporting the elongate element (50), the system being arranged in such a manner that the elongate element can be placed in a slot (10) of the head without the fingers (30) extending into the slot (10).

10. A system according to claim 9, **characterized in that** the fingers (30) extend from one end of an arm (22) of the tool (20).

11. A system according to any one of claims 1 to 10, **characterized in that** the tool (20) has an arm (24) suitable for applying the additional thrust (63).

12. A system according to any one of claims 1 to 11, **characterized in that** the additional thrust (63) is applied against a face (14) of the branch (8).

13. A system according to claim 12, **characterized in that** the tool (20) and the face (14) are shaped with complementary shapes to prevent the tool (20) sliding upwards or downwards relative to the head (6).

14. A system according to any one of claims 1 to 13, **characterized in that** the branch (8) is a first branch, and the head includes a second branch (8) extending facing the first branch.

15. A system according to claim 14, **characterized in that** the or each lateral thrust (43, 59) is applied in a direction away from the second branch (8).

16. A system according to claim 14 or claim 15, **characterized in that** the tool (20) is suitable for holding the branch (8) while leaving access to a slot (10) formed between the two branches.

17. A system according to any one of claims 1 to 16, **characterized in that** the tool (20) comprises two arms (22, 24) that are hinged to each other.

18. A system according to any one of claims 1 to 17, **characterized in that** it includes an anchor member suitable for being anchored in the spine, the head (6) being integral with said anchor member.

19. A system according to any one of claims 1 to 17, **characterized in that** it comprises an anchor member suitable for being anchored in the spine, the head (6) being suitable for fixing to the anchor member in different angular positions of the head relative to the anchor member.

## Patentansprüche

1. Spinale Osteosyntheseanordnung, umfassend:
- einen Kopf (6) eines Verankerungselements (4), der mindestens aus einem Flügel besteht, der eine Innenseite (12), eine Außenseite (14), eine Spitze (16) und zwei Seitenränder (18) aufweist, und
- ein Werkzeug (20) zur Befestigung des Kopfes (6) an einem spinalen Osteosynthesesystem, wobei das Werkzeug (20) derart ausgebildet ist, dass es den Kopf halten kann (6) durch seitliche Abstützung (43, 49) auf jedem der seitlichen Ränder (18) des Flügels und eine zusätzliche Abstützung (58) auf dem Flügel in entgegengesetzer Richtung zur seitlichen Abstützung, und **dadurch gekennzeichnet ist, dass** das Werkzeug (20) einen Arm (22) in einem Stück umfasst, der imstande ist, gleichzeitig die seitlichen Abstützungen (43, 49) und die zusätzliche Abstützung durchzuführen.

2. Anordnung nach Anspruch 1, **dadurch gekennzeichnet, dass** das Werkzeug (20) und die seitlichen Ränder (18) ausgebildet sind, um durch Formkomplementarität ein Gleiten des Werkzeugs nach oben und/oder nach unten des Kopfes (6) zu verhindern.

3. Anordnung nach Anspruch 1 oder 2, **dadurch gekennzeichnet, dass** das Werkzeug (20) zwei Reliefs (42) aufweist, um sich auf den Rändern (18) abzustützen.

4. Anordnung nach einem der Ansprüche 1 bis 3, **dadurch gekennzeichnet, dass** die seitlichen Ränder (18) zwei Hohlräume (36) aufweisen, auf denen das Werkzeug (20) zur Abstützung imstande ist.

5. Anordnung nach Anspruch 3, **dadurch gekennzeichnet, dass** die Hohlräume (136; 236) in Richtung einer Spitze (16) des Kopfes geöffnet sind.

6. Anordnung nach einem der Ansprüche 1 bis 5, **dadurch gekennzeichnet, dass** die seitliche Abstützung (58) auf einem oberen Rand (16) des Flügels (8) durchgeführt wird.

7. Anordnung nach Anspruch 6, **dadurch gekennzeichnet, dass** der Kontakt des Werkzeugs (20) mit dem oberen Rand (16) der Länge nach oder oberflächlich erfolgt.

8. Anordnung nach einem der Ansprüche 6 oder 7, **dadurch gekennzeichnet, dass** das Werkzeug (20) ein längliches Element (50) umfasst, dass sich quer in Bezug zu einer allgemeinen Richtung des Werkzeugs erstreckt und durch das es imstande ist, sich auf dem oberen Rand (16) abzustützen.

9. Anordnung nach Anspruch 8, **dadurch gekennzeichnet, dass** das Werkzeug (20) zwei Finger (30) umfasst, die das längliche Element (50) tragen, wobei die Anordnung derart ausgebildet ist, dass das längliche Element in einer Aufnahme (10) des Kopfes anordenbar ist, ohne dass sich die Finger (30) in die Aufnahme (10) erstrecken.

10. Anordnung nach Anspruch 9, **dadurch gekennzeichnet, dass** sich die Finger (30) an einem Ende eines Arms (22) des Werkzeugs (20) erstrecken.

11. Anordnung nach einem der Ansprüche 1 bis 10, **dadurch gekennzeichnet, dass** das Werkzeug (20) einen Arm (24) umfasst, der imstande ist, die zusätzliche Abstützung (63) zu gewährleisten.

12. Anordnung nach einem der Ansprüche 1 bis 11, **dadurch gekennzeichnet, dass** die zusätzliche Abstützung (63) auf einer Seite (14) des Flügels (8) durchgeführt wird.

13. Anordnung nach Anspruch 12, **dadurch gekennzeichnet, dass** das Werkzeug (20) und die Seite (14) ausgebildet sind, um durch Formkomplementarität ein Gleiten des Werkzeugs (20) nach oben und/oder nach unten des Kopfes (6) zu verhindern.

14. Anordnung nach einem der Ansprüche 1 bis 13, **dadurch gekennzeichnet, dass**, wobei der Flügel (8) ein erster Flügel ist, der Kopf einen zweiten Flügel (8) aufweist, der sich gegenüber dem ersten Flügel erstreckt.

15. Anordnung nach Anspruch 14, **dadurch gekennzeichnet, dass** die oder jede seitliche Abstützung (43, 59) in entgegengesetzter Richtung zum zweiten Flügel (8) durchgeführt wird.

16. Anordnung nach einem der Ansprüche 14 oder 15, **dadurch gekennzeichnet, dass** das Werkzeug (20) imstande ist, den Arm (8) zu halten und dabei den Zugriff auf eine Aufnahme (10) zwischen den zwei Flügeln zu ermöglichen.

17. Anordnung nach einem der Ansprüche 1 bis 16, **dadurch gekennzeichnet, dass** das Werkzeug (20) zwei Arme (22, 24) umfasst, die aneinander angelenkt sind.

18. Anordnung nach einem der Ansprüche 1 bis 17, **dadurch gekennzeichnet, dass** sie ein Verankerungselement umfasst, das imstande ist, in der Wirbelsäule verankert zu sein, wobei der Kopf (6) mit diesem Element in einem Stück ist.

19. Anordnung nach einem der Ansprüche 1 bis 17, **dadurch gekennzeichnet, dass** sie ein Verankerungselement umfasst, das imstande ist, in der Wirbelsäule verankert zu sein, wobei der Kopf (6) imstande ist, am Element gemäß verschiedener Winkelstellungen des Kopfes im Verhältnis zum Element befestigt zu sein.
